# EUROPÄISCHE PATENTANMELDUNG

(11) **EP 2 269 980 A1**
(43) Veröffentlichungstag der Anmeldung: **05.01.2011**
(21) Anmeldenummer: 08878506.8
(22) Anmeldetag: 24.12.2008
(51) Int. Cl.: C07C 279/10, C07C 277/08, C07K 5/068, C07K 5/11, A61K 38/05, A61K 38/07, A61K 31/16, A61P 25/28

(54) **CREATINAMIDE, VERFAHREN ZU IHRER HERSTELLUNG UND WIRKSTOFF ZUR AUSFÜHRUNG EINER NEUROPROTEKTIVEN AKTION**

(71) Anmelder: Vertex Closed Joint Stock Company, St.Petersburg 199026 (RU)
(72) Erfinder: KHROMOV, Alexej Nikolaevich, "deceased" (RU); BUROV, Sergej Vladimirovich, St.Petersburg 197198 (RU)
(74) Vertreter: Jeck, Anton
(86) Internationale Anmeldenummer: PCT/RU2008/000793
(87) Internationale Veröffentlichungsnummer: WO 2010/074591

(57) **Zusammenfassung**

Die Erfindung bezieht sich auf das Gebiet der pharmakologischen Chemie, nämlich auf neue biologisch aktive Substanzen und ihre Eigenschaften. Insbesondere betrifft die Erfindung Derivate von Kreatin - Stoffe mit einer Gesamtformel: NH=C(NH₂)-N(CH₃)-CH₂-CO-NH-R*X, wobei R einen Aminosäurerest oder substituierter Aminosäurerest und X eine niedermolekulare organische Säure oder Mineralsäure oder Wasser ist.

Die neuen Substanzen werden mittels Zusammenwirkung der guanidinierenden Agens mit Amiden von Sarkosin in polaren organischen Lösungsmitteln bei einer Temperatur von max. 50° C hergestellt.

Die neuen Zusammensetzungen weisen eine neuroprotektive Wirkung auf und können als aussichtsreiches Mittel zur Prophylaxe und Behandlung der Gehirnischämie angewendet werden.

## Beschreibung

Die Erfindung bezieht sich auf das Gebiet der pharmakologischen Chemie, nämlich auf neue biologisch aktive Substanzen und ihre Eigenschaften. Insbesondere betrifft die Erfindung Kreatin-Derivate mit der Gesamtformel: NH=C(NH₂)-N(CH₃)-CH₂-CO-NH-R*X, wobei R ein Aminosäurerest oder ein substituierter Aminosäurerest und X eine niedermolekulare organische Säure oder Mineralsäure oder Wasser ist.

Kreatin ist ein endogener Nährstoff. Er ist in unterschiedlichen Geweben von Säugetieren, z. B. in der Leber, in den Nieren, im Muskelgewebe, im Hirngewebe, im Blut sowohl in freier Form als auch als Kreatinphosphat immer vorhanden. Kreatin wird als Mittel zur Verbesserung des energetischen Gewebemetabolismus betrachtet. Es erhöht die energetische Reserve von ATP Adenosintriphosphat, vor allem in den Muskel- und Nervenzellen.

Kreatin wirkt reversibel mit Adenosintriphosphat (ATP (Adenosintriphosphat)) in den Mitochondrien der Zelle mit dem Ferment Kreatinkinase zusammen und bildet dabei Kreatinphosphat und Adenosindiphosphat (ADP). Die Funktion dieser Zusammenwirkung besteht darin, die Konzentration von ATP (Adenosintriphosphat) während seines intensiven Verbrauchs konstant zu halten. Andere Alternativen wie z. B. Glykolyse oder oxidative Phosphorylierung füllen die ATP (Adenosintriphosphat)-Vorräte viel langsamer auf. Beim Verbrauch von Adenosintriphosphat wird in der Zelle eine große Menge von ADP freigesetzt. Das verursacht die Orthophosphat-Übertragung vom Kreatinphosphat zum ADP und die Wiederherstellung des ursprünglichen Verhältnisses zwischen ATP (Adenosintriphosphat) und ADP. Dank der hohen ADP-Affinität der Kreatinkinase verläuft dieser Vorgang so lange, bis die Konzentration von Kreatinphosphat unter einige Dutzend µM fällt.

Während der Aufrechterhaltung des Membranpotentials, der Aktivierung von Metaboliten oder der Kontraktilitätsaktivität der Zelle stellt das Kreatinphosphat die Reserve des makroergischen Phosphats dar. Es gewährleistet einen bestimmten Stand von ATP bei der Erhöhung des Energieaufwands in der Zelle. Das heißt, es bringt den Orthophosphatrest zurück zum ADP. Neben Glykogen ist Kreatinphosphat eines der Hauptquellen des Umwandlungszyklus von hochenergetischen Phosphaten und ist somit in der oxidativen Glucose-Phosphorylierung beteiligt. Das stellt die Energieabgabe sicher, welche für die Funktion der Muskelgewebezellen einschließlich der Skelettmuskeln und des Herzmuskels erforderlich ist. Da das Kreatinphosphat das ATP (Adenosintriphosphat) mit höherer Geschwindigkeit regenerieren kann, als es unter Einsatz von Glykogen erreicht wird, steigert die Vergrößerung des Kreatingehalts in den Muskeln die Muskelvorräte an Kreatinphosphat, verbessert die Arbeitsfähigkeit (Ausdauerfähigkeit) der Muskeln und vergrößert die Muskelmasse.

Kreatinphosphat und Kreatin sind auch die allosterischen Regler der Zellenvorgänge. Es wurde nachgewiesen, dass die perorale Verabreichung von Kreatin den Gesamtgehalt von Kreatin im Körper erhöht. So führt die tägliche Einnahme von 20 bis 30 g Kreatin-Monohydrat im Laufe von einigen Tagen zur mehr als 20%iger Steigerung des Gesamtkreatingehalts in den menschlichen Skelettmuskeln. Diese Wirkung ist besonders attraktiv im Zusammenhang mit der Möglichkeit, Kreatin als Nahrungsergänzungsmittel zur Stärkung des Organismus und zur Erhöhung der Arbeitsfähigkeit, besonders bei Anwendung als Nahrungsergänzungsmittel in der Sportlerverpflegung, zu benutzen. So wird die Anwendung von Kreatin-Monohydrat in einer Tagesdosis von 15 g innerhalb von mindestens 2 Tagen zur Vergrößerung der Muskelmasse (WO 94/02127, 1994) eingesetzt. Zur Zeit wird Kreatin als Nahrungsergänzungsmittel empfohlen. Das ist für ältere Leute sowie für Vegetarier besonders wichtig, weil sich bei diesen Menschengruppen die Tendenz zur Abnahme des Kreatingehalts in den Muskeln sehr ausgeprägt erkennen lässt. Die Ergänzungsmittel werden als Trockenpulver, Flüssigkeit bzw. halbflüssig verwendet (WO 97/45026, 1997). Die hergestellten Mischungen/ Zusammensetzungen bleiben im Kühlschrank bei 4° C ziemlich lange stabil. Bei Raumtemperatur verfallen sie jedoch innerhalb einer Woche.

Neben der Anwendung in der Lebensmittelindustrie werden Kreatin und Kreatinphosphat weit verbreitet in der Medizin eingesetzt. So werden Kreatin, Kreatinphosphat und Cyclokreatin (US6706764, 2004) bei der Behandlung von Nervenerkrankungen, wie diabetische Neuropathie, toxische Neuropathie, Alzheimer-Krankheit, Parkinson-Krankheit, Insult usw., sowie bei Stoffwechselstörungen, wie Hyperglykämie und Zuckerkrankheit (US 6193973, 2001) empfohlen. Die perorale Anwendung von Kreatin ist für die Behandlung von Herz- und Ateminsuffizienz (WO/EP 97/06225, 1999), Asthma (US 6093746, 2000) offenbart. Die Verwendung von Kreatinphosphat ist bei der Behandlung von Herz-Kreislaufsystemerkrankungen indiziert und bei der Behandlung von Neubildungen als aussichtsreich (US 5219846, 1993) nachgewiesen.

Jedoch ist die Anwendung von Kreatin und Kreatinphosphat durch die geringe Löslichkeit und die Instabilität in Wassermedien bei physiologisch gerechten pH-Werten (RU 2295261, 2007) begrenzt.

Darüber hinaus wird Kreatin aus dem Magen-Darm-Kanal nur schlecht absorbiert. Der Absorptionsgrad beträgt 1 - 14 %. Das bedingt die Notwendigkeit der Anwendung von hohen Kreatindosen. Damit der Gebrauch von Kreatin wirksam ist, müssen die zur Zeit hergestellten Zusammensetzungen á 20 g pro Tag eingenommen werden. Zugleich kann die Einführung von hohen Kreatindosen neben der Steigerung der Therapie-Kosten auch negative Auswirkungen für den Körper verursachen, darunter Störung des Stickstoffwechsels, Magen-Darm-Störungen, Diarrhoe usw.

In diesem Zusammenhang erfährt die Herstellung von Kreatin-Derivaten ein sehr großes Interesse. Diese Derivate weisen eine längere Haltbarkeit und eine höhere Bioaktivität auf. Damit wird es ermöglicht, einerseits die Dosis der zu verwendenden Substanz zu verringern und andererseits neue Anwendungsgebiete zu entdecken.

Das größte Interesse rufen die Derivate von Kreatin und verschiedenen organischen Säuren hervor. So ist z. B. die Verwendung von Pyruvaten von Kreatin (US6166249, 2000; RU2114823, 1998) bekannt, um die Arbeitsfähigkeit zu steigern und das Gewicht zu verringern, sowie als Mittel für die Anpassung an die Sauerstoffinsuffizienz bei Ischämie, als Nahrungsergänzungsmittel, als Hautschutzmittel gegen Alterung und Einwirkung von Sonnenstrahlen (US7186754, 2007), als Heilmittel bei der Behandlung von Frauengeschlechtskrankheiten insbesondere von Dysmenorrhoe (US6503951, 2000).

Derivate von Kreatin und der Malon-, Malein-, Fumar-, Orotsäuren und Taurin (CN 10/249338, 2003; US 6861554, 2005; US 6166249, 2000; CA 10/740263, 2003) sind als Diättherapie in Form von Nahrungszusatzstoffen indiziert.. Kreatincitrat (US 2004077719, 2004) ist als nootropisches Mittel sowie für die Anwendung in kosmetischen Zusammensetzungen empfohlen. Unter anderen Kreatin-Derivaten sei auch Magnesiumsalz von Kreatinphosphat (CN 1709896, 2005) erwähnt, welches für die Einwirkung auf den Herzmuskel geeignet ist.

Der nächstliegende Stand der Technik gegenüber den angemeldeten Substanzen sind die Kreatin-Ether, z. B. Ethylether und Benzylester (WO 02/22135, 2002) und Zusammensetzungen auf deren Basis. Diese weisen im Vergleich zu Kreatin eine höhere Wasserlöslichkeit auf und dringen besser durch die Zellmembran hindurch. Die Pharmakokinetik der eigentlichen Ester von Kreatin wurde nicht erforscht. Es wurde jedoch angenommen, dass die genannten Derivate, wenn sie ins Blut gelangen, sich unter der Wirkung von Fermenten (Esterasen) in Kreatin umwandeln. Die Präparate auf der Basis von Kreatinestern werden als Nahrungs-ergänzungsmittel angewendet und peroral in Form von Lösungen, Emulsionen, Tabletten oder Kapseln eingenommen.

Der Nachteil der genannten Zusammensetzungen ist ihre unzureichende Haltbarkeit im Körper sowie die niedrige Bioäquivalenz. Deshalb wird der Gebrauch von Kreatinestern in fester Form bzw. in Pulvern sowie in erhöhten Tagesdosen bevorzugt.

Die durch diese Erfindung zu lösende technische Aufgabe ist die Entwicklung von neuen Kreatin-Derivaten, welche chemisch synthetisiert werden, eine höhere Haltbarkeit und ein breites biologisches Wirkungsspektrum aufweisen und insbesondere über eine neuroprotektive Wirkung verfügen.

Zur Zeit ist eine zahlreiche Gruppe von Heilmitteln bekannt, welche dazu fähig sind, eine spezifische Wirkung auf den energetischen Metabolismus des Hirngewebes auszuüben, die integrativen Funktionen des Gehirns zu aktivieren und die Widerstandsfähigkeit des Gehirns gegen Schadfaktoren zu erhöhen (M.D. Mashkovsky. Heilmittel, M., Medizin; Goodman E. Gilman's. The Pharmacological Basis of Therapeutics, 11 ed, McGraw-Hill, Medical Publishung Division, New York 2006; RU1746886, 1991, WO96/08527, 1996). Dazu gehören unter anderem:
- Derivate von Pyrrolidon (z.B., Piracetam), welche den Energiewechsel aktivieren;
- Präparate, welche die cholinergischen Vorgänge verstärken (z. B., Amiridinum, Tacrine, Gliatilin usw.);
- energetische GABA-Präparate (z. B. Präparate der Gamma-Aminobuttersäure, hopantenischen Säure, Picamilonum, Phenibutum);
- Aktivierungsenzyme des Krebs-Zyklus;
- Antioxidans und Membranschutzmittel (z. B. Mexidolum, Meclofenoxate, Pyritinol, Ubichinonum);
- Präparate mit komplexer metabolischer Wirkung (z. B. Vinpocetine), welche die Redoxvorgänge optimieren und zur Verbesserung des energetischen Metabolismus beitragen.

Die Nachteile der meisten genannten Stoffe sind das enge Wirkungsspektrum, häufige Kontraindikationen und eine geringe neuroprotektive Effizienz. Zugleich ist bekannt, dass es der Einsatz von wirksamen neuroprotektiven Mitteln in der Klinik ermöglichen könnte, den Anteil der «Kleininsulte» unter den ischämischen Gehirnkreislaufschädigungen zu erhöhen, die Abmessungen des Infarktbereichs zu vergrößern, die Zeitdauer des «Therapiefensters» zu verlängern und den Schutz gegen Reperfusionsverletzung zu sichern (Lancet, 2004, 363, 349-45).

Unter den Präparaten dieser Gruppe ist Actovegin am meisten verbreitet. Es ist der nächstliegende Stand der Technik gegenüber den angemeldeten Präparaten in Bezug auf die Wirkung. Actovegin enthält ein deproteinisierte Hämoderivat aus Kälberblut, welches in Form von Tabletten oder Injektionslösungen angewendet wird (Handbuch VIDAL, 2001, AstraPharmService, S. B-18)

### Wesen der Erfindung

Das technische Ergebnis wurde mittels Synthese der Kreatin-Derivate mit der NH=C(NH₂)-N(CH₃)-CH₂-CO-NH-R*X Gesamtformel erreicht,
wobei R ein Aminosäurerest oder ein substituierter Aminosäurerest und X organische Säure oder Mineralsäure oder Wasser ist.

Als Aminosäure können verschiedene aliphatische, aromatische und heteroaromatische L-Aminosäuren oder ihre Derivate, insbesondere Ester der Aminosäuren, Amide der Aminosäuren, Peptide u. a. m. angewendet werden. Als organische Säuren oder Mineralsäuren können pharmazeutisch geeignete niedermolekulare organische Säuren oder Mineralsäuren (Molekulargewicht in der Regel unter 300) angewendet werden, beispielsweise Essig-, Salz-, Zitronensäure u. a. m. Während der biologischen Experimente wurde festgestellt, dass die synthetisierten Kreatin-Amide gegenüber den bekannten Vergleichssubstanzen eine erhöhte Löslichkeit und Haltbarkeit in Wasserlösungen aufweisen. Das ermöglicht es, sie breiter als die Kreatin-Quelle im Körper anzuwenden.

Die Amide von Kreatin wurden mittels Zusammenwirkung von freien oder geschützten guanidinierenden Agentien mit Sarkosinamiden in polaren organischen Lösungsmitteln bei einer Temperatur von max. 50° C hergestellt. Die Durchführung der Synthese unter höheren Temperaturen verringert in der Regel die Ausbeute des Zielprodukts und wirkt sich negativ auf seine Bioaktivität durch Nebenreaktionen aus.

Die Wahl der konkreten Synthesebedingungen wird durch die Besonderheiten der genutzten Reagentien und des hergestellten Produkts festgelegt.

Die Derivate der Aminosäure können insbesondere unter Einsatz genormter chemischer Reaktionen, die in der Fachliteratur beschrieben sind (A.A. Gershkovich, V.K. Kibirev «Synthese der Peptide. Reagens und Verfahren». Kiev. «Naukova Dumka». 1987), oder unter Anwendung der Kreatin-Amide als Ausgangsstoff, die nach dem oben angeführten Verfahren erzeugt worden sind, hergestellt werden.

Die Analyse der chemischen Reinheit und die semipräparative Reinigung der Kreatin-Amide wurden nach dem Verfahren der HPLC-Chromatographie am Chromatograph-System Gold (Beckman) und den Säulen Phenomenex Luna C₁₈ (4,6 x 150 mm, 5 µm) für die analytische Chromatographie und Discovery C₁₈ (10 x 250 mm, 5 µm) für die semipräparative Chromatographie vorgenommen. Analysebedingungen: UV-Detektion bei 230 nm; Gradientenelution im System 0,1 % Trifluoressigsäurenlösung - Acetonitril bei Durchflussgeschwindigkeit von 1 ml/Min. - für die analytische Chromatographie; und Gradientenelution im System 0,1 % Trifluoressigsäurenlösung - Acetonitril bei Durchflußgeschwindigkeit 5 ml/Min. - für die semipräparative Chromatographie. Die Massenspektren wurden mit Hilfe von Flugzeitmassenspektrometern (Massenreflektron) MX-5303 mit einer lonenquelle vom Typ "Elektrospray" erfasst (Filiale des Instituts für Energieprobleme der chemischen Physik der Russischen Akademie für Wissenschaften (ΦNH 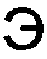 ΠXΦ PAH)).

### Ausführungsformen der Erfindungen

Die aussichtsreichste Anwendung von Kreatin-Amiden ist ihre Verwendung als Stoffe mit neuroprotektiver Wirkung. Die durchgeführten Versuche haben gezeigt: Bei der Einführung dieser Stoffe in den Körper in einer Dosis von 20 mg/kg und mehr wird die Verbesserung von kognitiven Funktionen beobachtet. Das macht die Kreatin-Amide zu einem aussichtsreichen Mittel zur Vorbeugung und Behandlung von ischämischen Gehirnschädigungen.

Die Kreatin-Amide können in den Körper sowohl alleine als auch als Bestandteil von Zusammensetzungen eingeführt werden, welche eine Mischung der Aktivsubstanzen mit Hilfsstoffen enthalten. Als Hilfsstoffe werden die durch Pharmakopöe genehmigten Stoffe verwendet, die die Bedingungen der Herstellung, der Lagerung oder der Anwendung des Heilmittels verbessern. Das können beispielsweise Lösungsmittel, Füllstoffe, Bindemittel, Auflockerer, Gleitmittel, filmbildende Mittel, Pigmente, Weichmachungsmittel, Verlängerungsmittel, Aromastoffe, Geschmackszutaten, Stabilisierungsmittel, Konservierungsstoffe u. a. m. sein.

So können z. B. als Hilfsstoffe solche Lösungsmittel, wie Wasser, Kalium-, Magnesium-, Zink-, Mangansalzbäder, physiologische Lösung, Sirupmassen; solche Füllstoffe, wie Crospovidone, Zucker und ihre Derivate, Polysaccharide und ihre Derivate, insbesondere Laktose, Saccharose, mikrokristalline Zellulose, Stärke, Glukose, Mannit, zyklische Dextrine, Alginate, Dextrin, Salze der organischen und mineralischen Säuren; Bindemittel, wie Wasser, Ethylalkohol, Zuckersirup, Stärkekleister, Lösungen von Zellulosederivaten, Povidone, Gelatine, Alginate u.a.m.; Auflockerer, wie Stärken, Crospovidone, Polysorbate, Natriumlaurylsulfat, Aerosil u. a. m.; Gleitmittel, wie Stärken, Talkum, Polyethylenglykol, Aerosil, Kalzium- und Magnesiumstearate, Stearinsäure, Natriumstearylfumarat u. a. m.; filmbildende Mittel, wie Alkylzellulosen und ihre Derivate u. a. m.; Weichmachungsmittel, wie Polysorbate, Glyzerin, Polyäthylenglykol, Propylenglykol, Dibutylphthalat, Glyzerintriazetat u. a. m. eingesetzt werden.

### Beispiel 1

### Synthese des Kreatinamids und der substituierten γ-Aminobuttersäure: Kreatinil-γaminobuttersäure-etheracetat

Eine Lösung von 0,66 g (2,08 mM) Trifluoroacetat-sarkosyl-γ-aminobuttersäure-ether in 1,5 ml Dimethylformamid wurde mit 0,94 ml (4,16 mM) Diisopropylamin und 0,9 g (2,08 mM) Benzolazimid-1-carboxamidin-tosylats ergänzt. Die Reaktionsmischung wurde im Laufe von 60 Stunden bei Raumtemperatur vermengt und mit n-Butanol und Wasser verdünnt. Das Lösungsmittel wurde eingedampft. Der Rückstand wurde aus Isopropylalkohol umkristallisiert und mit Hilfe der lonenaustauschchromatographie an einer Säule mit Sephadex SE C-25 in 0,002 M Pyridin-Acetatpuffer gereinigt. Die das Endprodukt enthaltenden Fraktionen wurden vereinigt, und das Lösungsmittel wurde eingedampft.
Ausbeute C₁₀H₂₀N₄O₃*CH₃COOH 0,3 g (59 %). Massenspektrum, gefunden: *m*/*z*: 245,17. Berechnet: *M* 244,15.

### Beispiel 2

### Synthese des Kreatinamids von substituiertem Phenylalanins: Kreatinil-L-phenylalaninamid-hydrat

Eine Lösung von 4 g (11,45 mM) Trifluoroacetat-amid-sarkosyl-L-phenylalanin in 30 ml Dimethylformamid wurde mit 3,2 ml (22,9 mM) Triethylamin und 5,3 g (11,45 mM) N,N'-Di-benzyloxycarbonyl-1-H-benzolazimid-1-carboxyamidin versetzt. Die Reaktionsmischung wurde im Laufe von 20 Stunden bei Raumtemperatur vermengt und mit 200 ml Ethylacetat verdünnt. Die organische Phase wurde mit 5 %iger Lösung von NaHCO₃, Wasser, 1 n HCl, Wasser gewaschen und 12 Stunden bei + 4° C gehalten. Der Niederschlag, das Amid Dibenzyloxycarbonyl-kreatinil-L-phenylalanin enthielt, wurde gefiltert, mit kaltem Ethylacetat gewaschen und getrocknet. Ausbeute 3,8 g (62 %).

Um die Schutzgruppe zu entfernen, wurden 3,8 g (6,96 mM) des Amids Dibenzyloxycarbonyl-kreatinil-phenylalanin in 150 ml der Mischung Dioxan - Wasser (9 : 1) aufgelöst und Pd auf Kohle im Laufe von 5 Stunden hydriert (Kontrolle mittels Dünnschichtchromatographie). Der Katalysator wurde abgefiltert. Das Lösungsmittel wurde eingedampft. Der Rückstand, welcher das Hydrat des Amids Kreatinil-L-phenylalanin enthielt, wurde aus Isopropylalkohol kristallisiert. Ausbeute C₁₃H₁₉N₅O₂*H₂O 1.2 g (43 %). Massenspektrum, gefunden: *m*/*z*: 278,15. Berechnet: *M* 277,15.

### Beispiel 3

### Synthese des Kreatinamids von substituiertem Phenylalanin: Kreatinil-L-phenylalaninamid-acetat

Das Amid Kreatinil-L-phenylalanin wurde vorher unter den Bedingungen aus Beispiel 2 hergestellt. Das Amid Kreatinil-L-phenylalanin wurde in Wasser aufgelöst und auf die Säule 25 x 100 mm, gefüllt mit Silicagel Lichroprep RP-18 (43-60 µm, Merck), aufgetragen, danach mit 0,2 %iger Essigsäure eluiert und aus der Lösung gewonnen.
Ausbeute: C₁₃H₁₉N₅O₂*CH₃COOH 0,72 g (60 %). Massenspektrum, gefunden: *m*/*z*: 278,15. Berechnet: *M* 277,15.

### Beispiel 4

### Synthese des Kreatinamids von substituiertem Glycin: Kreatinil-glycin-benzylestershydrochlorid

Eine Lösung von 2,5 g (7,07 mM) des Trifluoroacetats des Benzylesters Sarkosylglycin in 20 ml Dimethylformamid wurde mit 1,97 ml (14,14 mM) Triethylamin und 2,8 g (7,07 mM) N,N'-Di-tert-butyloxycarbonyl-1-H-benzolazimid-1-carboxamidin versetzt und innerhalb von 24 Stunden bei 20° C vermengt. Die Reaktionsmischung wurde mit 200 ml Ethylacetat verdünnt. Die organische Phase wurde mit 5 %iger Lösung NaH-CO₃, 1N H₂SO₄, Wasser gewaschen und über Na₂SO₄ getrocknet. Das Lösungsmittel wurde auf dem Rotorverdampfer eingedampft. Der Rückstand wurde aus der Mischung Ethylether - Petrolether kristallisiert. Ausbeute des Benzylesters Di-tert-Butyloxycarbonyl-kreatinil-glycin 2,3 g (67 %). R_{f} = 0,68 im System CHCl₃ : EtOAc : MeOH (20 : 10 : 3)

Um die Schutzgruppen zu entfernen, wurde durch die 2,3 g (4,78 mM) des Benzylesters Di-tert-butyloxycarbonyl-kreatinil-glycin in 70 ml trockenen Ethylethers enthaltende Lösung der Strom von trockener HCl bei 0° C und beim kräftigen Durchmischen im Laufe von 45 Minuten durchgelassen. Der Niederschlag wurde gefiltert, mit trockenem Ethylether gewaschen und im Exsikkator über NaOH getrocknet. Ausbeute C₁₃H₁₈N₄O₃- 0,3 g (20 %). Massenspektrum, gefunden: *m*/*z*: 279,14. Berechnet: *M* 278,14.

### Beispiel 5

### Synthese des Kreatinamid von substituiertem Tyrosin: Kreatinil-Tyrosinamid-Succzinat

Die Lösung von tert-Butyloxycarbonylsarkosin (4,49 g, 23,74 mM) und Hydroxybenzolazimid (3,21 g, 23,74 mM) in 30 ml Dimethylformamid wurde unter Kühlung im Eisbad und unter Umrühren mit einer Lösung von Dicylclohexylcarbodiimid (4,9 g, 23,74 mM) in 10 ml Dimethylformamid ergänzt. Nach 10 Minuten wurde die Reaktionsmischung mit Thyrosinmethylesterhydrochlorid (5 g, 21,58 mM) und Triethylamin (3,02 ml, 21,58 mM) ergänzt, danach im Laufe von 1 Stunde bei 0° C und im Laufe von 12 Stunden bei 20° C vermengt. Die Reaktionsmischung wurde mit 300 ml Ethylacetat verdünnt, mit 5 % NaHCO₃, 1N H₂SO₄, Wasser gewaschen und über Na₂SO₄ getrocknet. Das Lösungsmittel wurde auf dem Rotationsverdampfer eingedampft. R_{f} des Endproduktes beträgt 0,75 im System CHCl₃: EtOAc : MeOH (20 : 10 : 3). Ausbeute tert-Butyloxycarbonylsarkosyl-tyrosin-methylester - 6,2 g (78,5%).
6,2 g (16,92 mM) tert-Butyloxycarbonylsarkosyl-tyrosin-methylester wurden in 200 ml 6 M Ammoniaklösung in Methanol, gekühlt bis 0° C, aufgelöst. Der Kolben wurde dicht geschlossen. Die Lösung wurde innerhalb von 48 Stunden bei Raumtemperatur gehalten. Die Kontrolle des Reaktionsverlaufs erfolgte mittels Dünnschichtchromatographie im System CHCl₃: EtOAc:MeOH (20:10:3). R_{f} des Produkts beträgt 0,32. Das Lösungsmittel wurde eingedampft. Der Rückstand wurde mit Ethylether zerrieben, bis der trockene amorphe Rückstand gebildet wurde. Danach folgte die Trocknung im Vakuum. Ausbeute von tert-Butyloxycarbonylsarkosyltyrosinamid - 5,4 g (90 %).

5,4 g Butyloxycarbonylsarkosyltyrosinamid wurden in 20 ml Trifluoressigsäure aufgelöst und 15 Minuten lang bei Raumtemperatur gehalten. Das Lösungsmittel wurde auf dem Rotationsverdampfer bei 25° C eingedampft. Der Rückstand wurde aus dem Ethylether kristallisiert und im Vakuum getrocknet. Die Reinheit des hergestellten Produkts wurde mit Hilfe von HPLC-Chromatographie mit Phasenumkehr an der Säule Luna C-18 4,6 x 150 mm, 5 µm (Phenomenex) geprüft, und zwar unter Einsatz des linearen Gradienten von Acetonitril in Wasser. Dabei enthielt das Wasser 0,1 % der Orthophosphorsäure (0 - 20 % Acetonitril innerhalb von 20 Min.). Der Fluß betrug 1 ml/Min. Ausbeute von Trifluoroacetat-sarkosyl-tyrosinamid beträgt 5,4 g (96 %).

Die Lösung von Trifluoroacetat-sarkosyl-tyrosinamid (5,4 g, 14,8 mM) und Benzola-zimid-1-karboxamid-Tosylat (4,92 g, 14,8 mM) in 7 ml Dimethylformamid wurde unter Rühren mit N, N'-Diisopropylamin (5,06 ml, 29,6 mM) ergänzt. Gemäß den Ergebnissen der HPLC-Chromatographie betrug die Vollständigkeit des Reaktionsverlaufs nach 72 Stunden ca. 90 %. Das Lösungsmittel wurde mit einer Mischung Wasser: n-Butanol (2 : 3) eingedampft. Der Rückstand wurde in 20 ml Wasser aufgelöst und auf die Säule 25 x 150 mm mit Sephadex SE C-25 (Pharmacia Fine Chemicals) aufgetragen, die im 0,002 M Pyridin-Succzinatpuffer ausgeglichen wurde. Der Fluß betrug 2 ml/Min. Die weitere Auftrennung erfolgte mit dem Gradienten des Pyridin-Succinatpuffers innerhalb des Konzentrationsbereichs von 0,002 bis 0,5 M. Die Kreatiniltyrosinamidsukccinathaltigen Fraktionen wurden anhand der HPLC-Chromatographie mit Phasenumkehr analysiert, vereinigt und eingedampft. Die endgültige Reinigung erfolgte mittels Kristallisierung aus Isopropylalkohol. Die Reinheit des hergestellten Produkts wurde mit Hilfe der HPLC-Chromatographie mit Phasenumkehr an der Säule Luna C-18, 4,6 x 150 mm, 5 µm (Phenomenex) geprüft, und zwar unter Verwendung des linearen Gradienten von Acetonitril in Wasser, welches 0,1% Orthophosphorsäure (0 - 20% Acetonitril innerhalb von 20 Min) enthielt. Der Fluß betrug 1 ml/Min.

Die Ausbeute von Kreatiniltyrosinamidsucczinat betrug 2,7 g (51 %). Massenspektrum, gefunden: *m*/*z*: 292,27. Berechnet: *M* 292,29.

### Beispiel 6

### Synthese des Kreatinamid von substituiertem Glycin: Kreatinilglycinethylamid-Acetat.

Eine Lösung von tert-Butyloxycarbonylsarkosylglyzin-methylester (15 g, 54,88 mM) in 20 ml des trockenen Ethanol, gekühlt bis 0° C, wurde mit 10 ml Ethylamin ergänzt. Der Kolben wurde dicht verschlossen und bei Raumtemperatur stehen gelassen. Nach Kontrolle mittels Dünnschichtchromatographie war die Reaktion innerhalb von 48 Stunden vollständig. Das Lösungsmittel wurde auf dem Rotationsverdampfer eingedampft. R_{f} des Produktes betrug 0,45 im System CHCl₃: EtOAc : MeOH (20 : 10 : 3). Die Ausbeute von tert-Butyloxycarbonylsarkosyl-glycinethylamid betrug 14,7 g (98 %).

14,7 g (53,85 mM) tert-Butyloxycarbonylsarkosyl-glycinethylamid wurden in 40 ml Trifluoressigsäure aufgelöst und 15 Minuten lang bei Raumtemperatur gehalten. Das Lösungsmittel wurde auf dem Rotationsverdampfer bei 25° C eingedampft. Der Rückstand wurde aus 150 ml Ethylether kristallisiert und im Vakuum getrocknet. Die Reinheit des hergestellten Produkts wurde mit Hilfe von HPLC-Chromatographie mit Phasenumkehr an der Säule Zorbax ODS 250 × 4,6 mm, 5µm (DuPont) mit einer mobilen Phase geprüft, die 0,1% TFA (0,5 - 20 % Acetonitril innerhalb von 20 Minuten) enthielt. Die Ausbeute von Trifluoroacetatsarkosylglycinethylamid betrug 13,9 g (92 %).

Eine Lösung von Trifluoroacetat-sarkosylglycinethylamid (5 g, 17,3 mM) und Benzo-lazimid-1-karboxamid-Tosylat (5,78 g, 17,3 mM) in 10 ml Dimethylformamid wurde unter Umrühren mit N, N-Diisopropylamin (6 ml, 34,6 mM) ergänzt. Nach Kontrolle durch HPLC-Chromatographie mit Phasenumkehr an der Säule Zorbax ODS 250x4,6 mm, 5 µm (Gradient von Acetonitril 0-20 % innerhalb von 20 Minuten) verläuft die Reaktion nach 48 Stunden zu 90 %. Das Lösungsmittel wurde mit der Mischung Wasser - n-Butanol (2 : 3) eingedampft. Der Rückstand wurde in 40 ml von 20 % Isopropylalkohol in Wasser aufgelöst und auf die Säule 25 x 150 mm mit Sephadex SE C-25 (Pharmacia Fine Chemicals) aufgetragen, die in 0,002 M Pyridin-Acetatpuffer mit einem Isopropylalkohol-Gehalt von 20 % äquilibriert wurde. Danach erfolgte die Auftrennung mit einem Gradienten mit der Pufferkonzentration von 0,002 bis 0,25 M. Die Kreatinilglycinethylamid-Acetat-haltigen Fraktionen wurden nach dem HPLC-Chromatographie-Verfahren mit Phasenumkehr an der Säule Zorbax ODS 250x4,6 mm, 5 µm in der mobilen Phase analysiert, welche 0, 1 % TFA enthält (0 - 20 % Acetonitril innerhalb von 20 Minuten), vereinigt und eingedampft. Die endgültige Reinigung des Produkts erfolgte mittels Kristallisierung aus 10 ml Isopropylalkohol bei -5° C. Die Produktreinheit wurde mittels HPLC-Chromatographie mit Phasenumkehr an der Säule Zorbax ODS 250x4,6 mm, 5 µm mit einer mobilen Phase geprüft, die 0,1% TFA (0,5-20 % von Acetonitril innerhalb von 20 Minuten) enthielt. Die Ausbeute von Kreatinilglycinethylamid-Acetat betrug 2,7 g (56 %). Massenspektrum, gefunden: *m*/*z*: 214,27. Berechnet: *M* 214,25.

### Beispiel 7

### Synthese des Kreatinamids von substituiertem Phenylalanin: Kreatinilphenylalanylarginyl-glycin-etheracetat

Eine Lösung von Dicycohexylkarbodiimid-tert-butyloxycarbonyl-ornithin (7,9 g, 21,52 mM) und Hydroxybenzolazimid (2,91 g, 21,52 mM) in 20 ml Dimethylformamid wurde unter Kühlung im Eisbad und unter Umrühren mit einer Lösung von N, N'-Dicyclohexylcarbodiimid (4,44 g, 21,52 mM) in 10 ml Dimethylformamid ergänzt. Nach 10 Minuten wurde die Reaktionsmischung mit Glycindiethylether-hydrochlorid (3 g, 21,52 mM) und Triethylamin (3 ml, 21,52 mM) ergänzt und dann 1 Stunde lang bei 0° C und 20 Stunden lang bei 20° C vermengt. Die Reaktionsmischung wurde mit 300 ml Äthylazetat verdünnt, mit 5 %iger Lösung von NaHCO₃, 1N H₂SO₄, Wasser gewaschen und über Na₂SO₄ getrocknet. Das Lösungsmittel wurde auf dem Rotationsverdampfer eingedampft. Der Rückstand stellte einen kristallinen Stoff dar. Schmelztemperatur 113 - 115° C; R_{f} 0,89 im System CHCl₃: EtOAc : MeOH (20 : 10 : 3). Ausbeute: Dicyclohexylcarbodiimid-tert-butyloxycarbonyl-ornithyl-glycin-Ether - 8,6 g (88 %).

Eine Lösung von 8,6 g Dicyclohexylcarbodiimid-tert-butyloxycarbonyl-ornithyl-glycin-ether in 150 ml von über Mg destillierten Methanol wurde mit Palladium-Kohlenstoff ergänzt und im Laufe von 3 Stunden hydriert. Die Vollständigkeit der Reaktion wurde nach dem Dünnschichtchromatographie-Verfahren im System CHCl₃ : EtOAc : MeOH (20 : 10 : 3) geprüft. Das Lösungsmittel wurde auf dem Rotationsverdampfer eingedampft. Die Reinheit des hergestellten Produkts wurde mit Hilfe der Dünnschichtchromatographie im System EtOAc : n-BuOH : AcOH : H₂O (2 : 1 : 1 : 1) geprüft. R_{f} = 0,43.
Ausbeute: tert-Butyloxycarbonyl-ornithyl-glycin-Ether - 6 g (100 %).

Eine Lösung von Dicyclohexylkarbodiimidphenylalanin (6,24 g, 20,86 mM) und Hydroxybenzolazimid (2,82 g, 20,86 mM) in 20 ml Dimethylformamid wurde unter Kühlung im Eisbad und Rühren mit einer Lösung von Dicyclohexylkarbodiimid (4,3 g, 20,86 mM) in 10 ml Dimethylformamid ergänzt. 10 Minuten später wurde die Reaktionsmasse mit tert-Butyloxycarbonyl-ornithyl-glycin-diethylether (6 g, 18,92 mM) ergänzt und dann im Laufe von 1 Stunde bei 0⁰ C und im Laufe von 20 Stunden bei 20° C vermengt. Die Reaktionsmischung wurde mit 300 ml Ethylacetat verdünnt, mit 5 %iger Lösung von NaHCO₃, 1N H₂SO₄, Wasser gewaschen und über Na₂SO₄ getrocknet. Das Lösungsmittel wurde auf dem Rotationsverdampfer eingedampft. Das Produkt wurde beim Eindampfen kristallisiert. Schmelztemperatur 179 - 182° C; R_{f} 0,52 im System CHCl₃: EtOAc : MeOH (20 : 10 : 3).
Ausbeute: Dicyclohexylkarbodiimidphenylalanyl-ornithyl(tert-butyloxycarbonyl)-glycin-Ether 8,7 g (77 %).

Eine Lösung von 8,7 g Dicyclohexylcarbodiimid-phenylalanyl-ornithyl(tert-butyloxycarbonyl)-glycin-ether in 150 ml des über Mg destillierten Methanols wurde mit Palladium-Kohlenstoff ergänzt und im Laufe von 3 Stunden hydriert. Die Vollständigkeit der Reaktion wurde mittels Dünnschichtchromatographie im System CHCl₃ : EtOAc : MeOH (20 : 10 : 3) geprüft. Das Lösungsmittel wurde auf dem Rotorverdampfer eingedampft. Die Reinheit des hergestellten Produkts wurde mittels Dünnschichtchromatographie im System EtOAc : n-BuOH : AcOH : H₂O (2 : 1 : 1 : 1) geprüft. R_{f} = 0,27.
Ausbeute: Phenylalanyl-ornithyl(tert-Butyloxycarbonyl)-glycin-ether - 6,58 g (100 % ).

Eine Lösung von tert-Butyloxycarbonylsarkosin (3,78 g, 20 mM) und Hydroxybenzolazimid (2,7 g, 20,00 mM) in 20 ml Dimethylformamid wurde unter Kühlung im Eisbad und unter Rühren mit einer Lösung von Dicyclohexylcarbodiimid (4,12 g, 20,00 mM) in 10 ml Dimethylformamid ergänzt. Nach 10 Minuten wurde die Reaktionsmischung mit Phenylalanyl-ornithyl(tert-butyloxycarbonyl)-glycin-ether (6,58 g, 14,2 mM) ergänzt und im Laufe von 1 Stunde bei 0° C und im Laufe von 20 Stunden bei 20° C vermengt. Die Reaktionsmischung wurde mit 300 ml Ethylacetat verdünnt, mit 5 %iger Lösung von NaHCO₃, 1N H₂SO₄, Wasser gewaschen. Die Lösung wurde gekühlt und 3 Stunden bei 4° C gehalten. Das Produkt fiel aus. Der Niederschlag wurde gefiltert, mit Ether gewaschen und im Vakuum getrocknet. Schmelztemperatur 189 -192°C; R_{f} 0,40 im System CHCl₃ : EtOAc : MeOH (20 : 10 : 3).
Ausbeute:
tert-Butyloxycarbonylsarkosyl-phenylalanyl-ornithyl(tert-butyloxycarbonyl)-glycin-ether - 6,5 g (72 %).

6,5 g tert-Butyloxycarbonylsarkosyl-phenylalanyl-ornithyl(tert-butyloxycarbonyl)-glycin-ether wurden in 40 ml Trifluoressigsäure aufgelöst und 15 Minuten lang bei Raumtemperatur belassen. Das Lösungsmittel wurde auf dem Rotationsverdampfer bei 25° C eingedampft. Der Rückstand wurde aus 250 ml Ethylether kristallisiert und im Vakuum getrocknet. Die Reinheit des hergestellten Produkts wurde anhand der HPLC-Chromatographie mit Phasenumkehr an der Säule Phenomenex Luna C-18, 5µ, 4,6x150 mm unter Einsatz des linearen Gradienten von Acetonitril in Wasser geprüft, wobei das Wasser 0,1 % der Trifluoressigsäure (7 - 27 % Acetonitril innerhalb von 20 Minuten) enthielt. Der Fluß betrug 1 ml/Min.
Ausbeute: Trifluoroacetat-sarkosyl-phenylalanyl-ornithyl-glycin-ether - 6,5 g (96 %).

Eine Lösung von Trifluoroacetat-sarkosyl-phenylalanyl-ornithyl-glycin-ether (2 g, 3,02 mM) in 5 ml von Wasser wurde auf die Säule 15x 100 mm mit Amberlit IRA-67 ("Sigma") in der OH- -Form aufgetragen. Die Säule wurde mit Wasser durchgespült.

Die Fraktionen mit einem pH-Wert von größer als 7 wurden vereinigt, eingedampft und getrocknet nach dem Verfahren des dreimaligen Abdestillierens mit Isopropylalkohol. Das hergestellte sarkosyl-phenylalanyl-ornithyl-glycin-ether wurde in 5 ml Dimethylformamid aufgelöst, mit 0,5 g LiCl (Merck) zur Verbesserung der Löslichkeit ergänzt. Danach wurde das N, N' - Diisopropylamin (1,05 ml, 6,04 mM) und Benzo-lazimid-1-carboxamid-Tosylat (2,01 g, 6,04 mM) zugegeben, mit 5 ml Dimethylformamid ergänzt und innerhalb von 72 Stunden gehalten. Nach Prüfung durch HPLC-Chromatographie mit Phasenumkehr an der Säule Phenomenex Luna C-18, 5µ, 4,6x150 mm, linearer Gradient von Acetonitril in Wasser, mit einem Trifluoressigsäure-Gehalt von 0,1% (7 - 27 % Acetonitril innerhalb von 20 Minuten, Fluß 1 ml/Min.), verläuft die Reaktion fast vollständig, d. h. zu mehr als 90 % in 40 Stunden. Das Lösungsmittel wurde mit der Mischung Wasser - n-Butanol (2 : 3) eingedampft. Der Rückstand wurde in 20 ml Wasser aufgelöst und auf die Säule 25 x 150 mm mit Sephadex SE C-25 (Pharmacia Fine Chemicals) aufgetragen, ausgeglichen in 0,002 M Pyridin-Acetatpuffer. Die Säule wurde mit 400 ml 0,002 M Puffer (Fluß 2 ml/Min.) gespült. Die Trennung erfolgte innerhalb des Gradienten 0,002-0,5 M des Pyridin-Acetatpuffers. Die Kreatinil-phenylalanyl-arginyl-glycin-Diethylether-acetat-haltigen Fraktionen wurden vereinigt und eingedampft. Die endgültige Reinigung des Produkts erfolgte mittels Kristallisierung aus 20 ml Isopropylalkohol bei Raumtemperatur. Die Reinheit des hergestellten Produkts wurde mittels der HPLC-Chromatographie mit Phasenumkehr an der Säule Luna C-18, 5 µm, 4,6x150 mm (Phenomenex) unter Einsatz des linearen Gradienten von Acetonitril in Wasser geprüft, mit 0,1 %igem Orthophosphorsäure-Gehalt (8 - 28 % von Acetonitril innerhalb von 20 Minuten). Die Strömungsgeschwindigkeit betrug 1 ml/Min.
Ausbeute: Kreatinil-phenylalanyl-arginyl-glycin-diethylether-acetat 0,72 g (37 %). Massenspektrum, gefunden: *m*/*z*: 520,60. Berechnet: M 520,61.

### Beispiel 8

### Synthese des Kreatinamids von unsubstituiertem Phenylalanin: Kreatinilphenylalanin-chlorhydrat

Eine Lösung von tert-Butyloxycarbonylsarkosin (4 g, 21,14 mM) und Hydroxybenzotriazol (2,86 g, 21,14 mM) wurde in 30 ml Dimethylformamid unter Kühlung im Eisbad und unter Rühren mit einer Lösung von Dicyklohexylcarbodiimid (4,36 g, 21,14 mM) in 10 ml Dimethylformamid ergänzt. Nach 10 Minuten wurden in die Reaktionsmischung Phenylalaninbenzylester-n-toluol-sulfonsäure (8,13 g, 19,03 mM) und Triethylamin (2,7 ml, 19,03 mM) gegeben. Die Mischung wurde innerhalb von 1 Stunde bei 0° C und innerhalb von 20 Stunden bei 20° C vermengt. Der N,N'-Dicyclohexylharnstoff wurde gefiltert. Die Reaktionsmischung wurde mit 200 ml Ethylacetat verdünnt, mit 5 %iger Lösung von NaHCO₃, 1N H₂SO₄, Wasser gewaschen und über Na₂SO₄ getrocknet. Das Lösungsmittel wurde auf dem Rotationsverdampfer eingedampft. R_{f} der Grundsubstanz beträgt 0,85 im System CHCl₃: EtOAc : MeOH (20 : 10 : 3).
Ausbeute: tert-Butyloxycarbonylsarkosylphenylalanin-benzylester - 7,3 g (90 %).

7,3 g (17,15 mM) tert-Butyloxycarbonylsarkosylphenylalanin-benzylester wurden in 20 ml 65 %iger Trifluoressigsäure in CH₂Cl₂ aufgelöst und 30 Minuten lang bei Raumtemperatur belassen. Das Lösungsmittel wurde auf dem Rotationsverdampfer bei 25° C eingedampft. Der Rückstand wurde aus 200 ml Ethylethers kristallisiert und im Vakuum getrocknet. Die Reinheit des hergestellten Produkts wurde mittels HPLC-Chromatographie mit Phasenumkehr an der Säule Phenomenex Luna C-18, 5 µm, 4,6 x 150 mm (20 - 40 % von Acetonitril innerhalb von 20 Minuten) geprüft.
Ausbeute: Sarkosylphenylalaninbenzylester-trifluoroacetat - 6 g (80 %).

Eine Lösung von Sarkosylphenylalanin-benzylestertrifluoroacetats (6 g, 13,62 mM) und Benzolazimid-1-karboxyamid-tosylat (4,54 g, 13,62 mM) in 7 ml Dimethylformamid wurde unter Rühren mit N,N'-Diisopropylamin (4,74 ml, 27,24 mM) ergänzt. Nach Prüfung durch HPLC-Chromatographie mit Phasenumkehr an der Säule Phenomenex Luna C-18, 5 µm, 4,6 x 150 mm (20 - 40 % von Acetonitril innerhalb von 20 Minuten) verlief die Reaktion nach 72 Stunden praktisch vollständig. Das Lösungsmittel wurde mit der Mischung Wasser - n-Butanol (2 : 3) eingedampft. Der Rückstand wurde in 80 ml des 30 %igen wasserhaltigen Isopropylalkohols aufgelöst und auf die Säule 25 x 150 mm mit Sephadex SE C-25 (Pharmacia Fine Chemicals), äquilibriert mit 0,002 M Pyridin-Azetatpuffer mit 20 % Isopropylalkohol-Gehalt aufgetragen. Die Säule wurde mit 400 ml des Anfangspuffers (Fluß 2 ml/Min.) gewaschen. Die Trennung erfolgte mit einem Gradienten 0,002 - 0,5 M von Pyridin-Acetatpuffer mit 20 % Isopropylalkohol-Gehalt.

Die Kreatinilphenylalaninbenzylesteracetat-haltigen Fraktionen wurden mittels HPLC-Chromatographie mit Phasenumkehr an der Säule Phenomenex Luna C-18, 5 µm, 4,6 x 150 mm (20 - 40 % von Acetonitril innerhalb von 20 Minuten), vereinigt und eingedampft. Ausbeute: Kreatinilphenylamin-benzylesteracetat - 2,54 g (41 %).

2,54 g (5,93 mM) Kreatinilphenylamin-benzylester-acetat wurden in 80 ml Methanol aufgelöst und über Pd-Kohlenstoff hydriert. Im Verlauf der Reaktion fiel das Produkt aus. 4 Stunden später war das Hydrieren nach den Ergebnissen der Dünnschichtchromatographie im System ACN: H₂O:AcOH (7 : 1 : 1) praktisch vollständig abgeschlossen. Der Niederschlag wurde zusammen mit dem Katalysator gefiltert, mit Methanol gewaschen und in 200 ml 0,01 M HCl in 40 %iger Wasserlösung von Methanol aufgelöst. Der Katalysator wurde gefiltert. Das Filtrat wurde eingedampft. Der Rückstand wurde aus 10 ml Isopropylalkohol kristallisiert. Die Reinheit des hergestellten Produkts wurde mittels HPLC-Chromatographie mit Phasenumkehr an der Säule Luna C-18, 5 µm, 4,6 x 150 mm, Phenomenex (5 - 25 % von Azetonitril innerhalb von 20 Minuten) analysiert.
Ausbeute: 1,1 g (69 %). Massenspektrum, gefunden: *m*/*z*: 278,27. Berechnet: M 278,29.

### Beispiel 9

### Untersuchung zur Haltbarkeit von Kreatin-Amiden in Wasserlösung und im Blutplasma

Die Untersuchung der Haltbarkeit von Kreatin-Amiden in Wasserlösung sowie im Blutplasma des Menschen und der Ratte wurde nach dem Verfahren der HPLC-Chromatographie mit Phasenumkehr unter Einsatz des chromatographischen Systems Beckman System Gold (USA) in folgender Bestückung durchgeführt: Programmable Solvent Module 126, Manual Injector Rheodyne 7725i, Programmable Detector Module 166, komplett mit Steuerungs- und Datenverarbeitungssoftware Beckman System Gold Chromatography Software. Die Analyse erfolgte an der Säule Luna 5µ C18(2) 100 Å 150 x 4,6 mm ( Phenomenex, USA) mit der Vorsäule Guard Cartridge C18 bei einer Durchflussgeschwindigkeit von 1 ml/Min. innerhalb eines linearen Acetonitril-Gradienten: für das Vergleichserzeugnis aus Beispiel Nr. 1 von 5 % bis 25 % Acetonitril innerhalb von 20 Minuten (Puffer A: 0,1 % H₃PO₄- H₂O, Puffer B: 0,1 % H₃PO₄ - Acetonitril); für die Substanz aus den Beispielen 2 - 8 von 3 % bis 23 % Acetonitril innerhalb von 20 Minuten. Die Detektion erfolgte mit einer Wellenlänge von 220 nm. Die Dosierung der Proben auf die Säule erfolgte mit der Schleife 20 µl.

Für die Zubereitung der Lösungen der zu untersuchenden Substanzen wurde auf der Analysenwaage je eine präzise Einwaage von jedem Amid abgewogen. Jede Einwaage wurde mit der berechneten Menge destillierten Wassers ergänzt, um die Konzentration von 2 mg/ml zu erreichen. Ein Teil der Lösung wurde zehnfach verdünnt. Sofort danach wurde die Analyse der Probe vorgenommen. Danach wurde diese Lösung bei Raumtemperatur gehalten. 3 Stunden später wurde die Analyse noch einmal wiederholt. Nach den Ergebnissen dieser Auswertungen nahm die Fläche der Spitzen beider Präparate nach 3 Stunden unwesentlich (unter 3 %) ab. Die Bildung von neuen Spitzen wurde nicht eingeengt.

Zur Untersuchung der Haltbarkeit der Kreatin-Amide wurden 200 µl der Wasserlösung des Ausgangsamids mit einer Konzentration von 2 - 3 mg/ml mit 1 ml Wasser oder Blutplasma ergänzt und geschüttelt. Es wurde sofort eine 200-µl-Probe entnommen und die Anfangskonzentration von Kreatin-Amid analysiert. Danach wurde die Lösung in den Vibrationsthermostat unter eine Temperatur von 37° C gesetzt. Dieser Lösung wurde je ein Aliquot von 200 µl nach jeweils 0,5, 1 und 3 Stunden der Thermostatisierung entnommen. Die entnommene Probe wurde mit 20 µl der 10 %i-gen Lösung Trichloressigsäure ergänzt und 15 Minuten unter einer Temperatur von Minus 24° C belassen, danach bei 6000 g innerhalb von 5 Minuten zentrifugiert, um das Plasmaprotein abzusetzen. Der Überstand wurde entnommen und analysiert. Um über die Haltbarkeit der Präparate zu urteilen, wurden die Spitzenflächen der jeweiligen Zusammensetzung am Anfang des Versuchs und nach den gewählten Zeitabständen verglichen (Tabellen 1-2).

**Tabelle 1 - Haltbarkeit der Kreatin-Vergleichserzeugnisse im Blutplasma des Menschen**

| Präparat | Haltbarkeit im Blutplasma des Menschen | | | | |
|---|---|---|---|---|---|
| | 0 Std. | 0,5 Std. | 0,75 Std. | 1 Std. | 3 Std. |
| Nach Beispiel 1 | 100 % | 99 % | - | 103 % | 104 % |
| Nach Beispiel 2 | 100 % | 99 % | - | 99 % | 100 % |
| Nach Beispiel 3 | 100 % | 100 % | - | 100 % | 100 % |
| Nach Beispiel 4 | 100 % | 98 % | - | 100 % | 99 % |
| Nach Beispiel 5 | 100 % | 98 % | - | 97 % | 93 % |
| Nach Beispiel 6 | 100 % | 97 % | - | 95 % | 95 % |
| Nach Beispiel 7 | 100 % | 94 % | - | 90 % | 85 % |
| Nach Beispiel 8 | 100 % | 99% | - | 98 % | 97 % |
| Kreatin-Benzylester | 100 % | - | 53 % | - | 46 % |

**Tabelle 2 - Haltbarkeit der Kreatin-Vergleichserzeugnisse im Blutplasma der Ratte**

| Präparat | Haltbarkeit im Blutplasma der Ratte | | | |
|---|---|---|---|---|
| | 0 Std. | 0,5 Std. | 1 Std. | 3 Std. |
| Nach Beispiel 1 | 100 % | 96 % | 98 % | 99 % |
| Nach Beispiel 2 | 100 % | 100 % | 97 % | 98 % |
| Nach Beispiel 3 | 100 % | 96 % | 98 % | 99 % |
| Nach Beispiel 4 | 100 % | 99 % | 99 % | 96 % |
| Nach Beispiel 5 | 100 % | 98 % | 97 % | 93 % |
| Nach Beispiel 6 | 100 % | 97 % | 97 % | 95 % |
| Nach Beispiel 7 | 100 % | 93 % | 92 % | 85 % |
| Nach Beispiel 8 | 100 % | 98 % | 98 % | 95 % |

Wie aus den oben angeführten Daten hervorgeht, weisen die Kreatin-Amide eine hohe Haltbarkeit im Plasma sowohl des Menschen als auch der Ratten auf. Ihre Konzentration blieb im Laufe von 3 Stunden so gut wie unverändert. Dagegen nahm die Konzentration des vergleichbaren Präparats (Kreatin-Benzylester) im Blutplasma des Menschen innerhalb von 0,75 Stunden in allen Fällen bis zu 53 % ab.

### Beispiel 10

### Neotropische Wirkung von Kreatin-Amiden

Die Untersuchung des Einflusses von Kreatin-Amiden in Bezug auf ihre Fähigkeit, eine neuroprotektive Wirkung auf das Hirngewebe auszuüben, wurde am Modell der Herd- und globalen zerebralen Ischämie der Ratten durchgeführt.

Die globale zerebrale lschämie wurde bei Ratten (Männchen) S-D iniziiert. Die Ratten wurden vorher mit Nembutal betäubt, indem die beiden Kopfschlagadern für 12 Minuten mit gleichzeitiger kontrollierbarer Hypotension abgeklemmt wurden (45 mm Hg).

Die intrazerebroentrikuläre Kanüle wurde stereotaxisch in die linke laterale zerebrale Kammer unter Einsatz der Ketamine-Betäubung eingeführt und an die osmotische Minipumpe Alzet (AP) angeschlossen (Modell 1002, Lösungsfördergeschwindigkeit 0,25 µl/St.), welche vorher mit der Versuchslösung gefüllt und subkutan zwischen den Schulterblättern angeordnet wurde

Die zu untersuchenden Präparate wurden intrazerebroentrikulär (a, b), oder peroral (a) in den Dosen von je 20 mg/kg Gewicht eingeführt; und zwar in folgenden Ausführungsformen des Versuchsablaufs:
(a) - Anfang: 5 Tage vor der Ischämie und dann innerhalb von 7 Tagen nach der Ischämie (Gütebeurteilung des Präparats zur Vorbeugung und Behandlung von Ischämie);
(b) - Anfang: 30 Minuten nach der Induktion der Ischämie und innerhalb von 7 Tagen danach (Gütebeurteilung des Präparats für die Behandlung von Ischämie);
(c) - Anfang: 1 Stunde vor der Ischämie (Gütebeurteilung des Präparats für die Vorbeugung und Behandlung von Ischämie);

Am 7. Tag nach der Ischämie wurden die Ratten mittels Dekapitation getötet, das Gehirn wurde entfernt und für 12 - 24 Stunden in 2 %igen Paraformaldehyd und dann in 96 %iges Äthanol eingebracht, für die "Blindanalyse" verschlüsselt und morphologisch untersucht. Der Schädigungsgrad des Gehirns wurde nach der Anzahl der «zusammengeballten» Neuronen an unterschiedlichen Stellen des Gehirns beurteilt.

Im Allgemeinen wurde der Schluss über die Wirksamkeit der Präparate auf der Grundlage der Untersuchung ihres Einflusses auf die Verhaltensreaktionen der Tiere im Morris-Wasserlabyrinth (Morris Water Maze) sowie aufgrund der Analyse der morphologischen Änderungen des Hirngewebes bei Einführung von Kreatin-Amiden im Hintergrund der globalen sowie der zerebralen Herdischämie gezogen.

Angaben über die Verhaltenstests im Morris-Wasserlabyrinth bei intraperitonaler Einführung des Präparats.

Für die Untersuchung wurden folgende Zusammensetzungen des Präparats benutzt (Tabelle 3).

**Tabelle 3 - Zusammensetzungen für die biologische Untersuchung**

| **Nr.** | **Komponentenbezeichnung** | **Menge, g** |
|---|---|---|
| **Zusammensetzung 1.** | | |
| | Kreatin-Amid nach Beispiel 1 | 20,0 |
| | Natriumchlorid | 9,0 |
| **1** | Nipaginum | 1,0 |
| | Ätznatron 0,1 M Lösung | bis pH 7,0 |
| | Wasser | auf 1 l |

| **Zusammensetzung 2.** | | |
|---|---|---|
| **2** | Kreatin-Amid nach Beispiel 2 | 10,0 |
| | Magnesiumsulfat | 10,0 |
| | Nipaginum | 1,0 |
| | Ätznatron 0,1 M, Lösung | bis pH 7,2 |
| | Wasser | auf 1 l |

| **Zusammensetzung 3.** | | |
|---|---|---|
| | Kreatin-Amid nach Beispiel 1 | 30,0 |
| | Bernsteinsäure | 0,5 |
| **3** | Nipaginum | 1,0 |
| | Ätzkali 0,1 M, Lösung | bis pH 7,2 |
| | Wasser | auf zu 1 l |

| **Zusammensetzung 4.** | | |
|---|---|---|
| | Kreatin-Amid nach Beispiel 3 | 0,35 |
| | Mikrokristalline Zellulose | 0,0769 |
| **4** | Crospovidone | 0,022 |
| | Kalziumphosphat-Dihydrat | 0,1000 |
| | Natriumstearylfumarat | 0,1000 |
| | Insgesamt: | 0,65 |

| **Zusammensetzung 5.** | | |
|---|---|---|
| | Kreatin-Amid nach Beispiel 4 | 0,35 |
| | Crospovidone | 0,017 |
| | Laktose | 0,003 |
| **5** | Stearinsäure | 0,002 |
| | Kaliumzitrat | 0,002 |
| | Aerosil | 0,001 |
| | Insgesamt: | 0,375 |

| **Zusammensetzung 6.** | | |
|---|---|---|
| | Kreatin-Amid nach Beispiel 4 | 0,35 |
| **6** | Mannit | 0,006 |
| | Aerosil | 0,001 |
| | Insgesamt: | 0,357 |

| **Zusammensetzung 7.** | | |
|---|---|---|
| **7** | Actovegin (wirkungsähnlich) | 0,35 |
| | Mikrokristalline Zellulose | 0,0769 |
| | Crospovidone | 0,022 |
| | Kalziumphosphat-Dihydrat | 0,100 |
| | Natriumstearylfumarat | 0,100 |
| | Insgesamt: | 0,65 |

| **Zusammensetzung 8.** | | |
|---|---|---|
| | Kreatin-Amid nach Beispiel 5 | 2,0 |
| | Natriumchlorid | 9,0 |
| **8** | Nipaginum | 1,0 |
| | Ätznatron 0,1 M, Lösung | bis pH 7,2 |
| | Wasser | bis zu 1 l |

| **Zusammensetzung 9.** | | |
|---|---|---|
| | Kreatin-Amid nach Beispiel 7 | 2,0 |
| | Natriumchlorid | 9,0 |
| **9** | Nipaginum | 1,0 |
| | Ätznatron 0,1 M, Lösung | bis pH 7,2 |
| | Wasser | bis zu 1 l |

| **Zusammensetzung 10.** | | |
|---|---|---|
| | Kreatin-Amid nach Beispiel 6 | 20,0 |
| | Natriumchlorid | 9,0 |
| **10** | Nipaginum | 1,0 |
| | Ätznatron 0,1 M, Lösung | bis pH 7,2 |
| | Wasser | bis zu 1 l |

Bei der Analyse der Wirksamkeit in Bezug auf Störungen des energetischen Gewebe-Metabolismus infolge der fokalen Gehirnischämie wurden die Lerndaten im Morris-Wasserlabyrinth für die Rattengruppen mit intraperitonalen Injektionen und peroraler Einführung der Präparate (n=7), der negativen Kontrolle mit intraperitonaler Einführung der physiologischen Kochsalzlösung (n=13), der positiven Kontrolle (n=5) und der falsch operierten Tiere (FO) (n=5) verglichen.

Die statistische Analyse (ANOVA-Verfahren mit wiederholten Messungen) hat gezeigt, dass die Gruppen sich in Bezug auf den Lernverlauf wesentlich unterscheiden. Das Lernen wurde bei folgenden Gruppen der Tiere beobachtet: Versuchsgruppe (Einführung des Präparats), Gruppe der positiven Kontrolle und der falsch operierten Tiere. Die PPM per se sperrte das Lernen völlig ab. Die mit dem Präparat behandelten Ratten verbrauchten statistisch weniger Zeit für die Suche nach der Plattform als die Ratten aus der Gruppe der negativen Kontrolle innerhalb der zwei letzten Lerntage (p < 0,02 und p < 0,03, dementsprechend; post hoc LSD Fisher's Test) und unterschieden sich nicht von der Gruppe der positiven Kontrolle und der falsch operierten Tiere (Tabelle 4).

**Tabelle 4 - Angaben der Verhaltenstests der Ratten im Morris-Wasserlabyrinth**

| Lerntag / Plattformsuchzeit, Sek. | | | | |
|---|---|---|---|---|
| 1 | 2 | 3 | 4 | 5 |
| Zusammensetzung 1, intrazerebroentrikuläre Einführung, Modell des Experiments a) | | | | |
| 52±5 | 44±3 | 42±3 | 40±2 | 35±3 |

| Zusammensetzung 2, intrazerebroentrikuläre Einführung, Modell des Experiments b) | | | | |
|---|---|---|---|---|
| 50±5 | 43±3 | 43±3 | 39±2 | 34±3 |

| Zusammensetzung 3, intrazerebroentrikuläre Einführung, Modell des Experiments b) | | | | |
|---|---|---|---|---|
| 47±4 | 45±3 | 41±4 | 39±3 | 35±3 |

| Zusammensetzung 4, peroral, Modell des Experiments a) | | | | |
|---|---|---|---|---|
| 55±3 | 45±5 | 42±3 | 41±3 | 34±4 |

| Zusammensetzung 5, peroral, Modell des Experiments a) | | | | |
|---|---|---|---|---|
| 52±3 | 45±5 | 40±3 | 40±2 | 33±4 |

| Zusammensetzung 6, peroral, Modell des Experiments a) | | | | |
|---|---|---|---|---|
| 52±3 | 46±5 | 41±3 | 40±2 | 32±4 |

| Zusammensetzung 7, peroral, Modell des Experiments a) | | | | |
|---|---|---|---|---|
| 52±3 | 55±5 | 53±3 | 53±2 | 50±5 |

| Herdischämie ohne Behandlung | | | | |
|---|---|---|---|---|
| 52±4 | 54±5 | 53±5 | 53±3 | 52±4 |

| Falsch operierte Tiere | | | | |
|---|---|---|---|---|
| 32±12 | 29±15 | 27±12 | 18±7 | 12±5 |

| Herdischämie + Hypothermie | | | | |
|---|---|---|---|---|
| 62±4 | 57±5 | 55±7 | 45±5 | 35±4 |

Die Anwendung der Kreatin-Amide-haltigen Zusammensetzungen verminderte wesentlich die Latenz der Bewegungsauslösung früh nach der Auslösung der PPM - nach 1 und 3 Tag(en). In Bezug auf diesen Effekt kommt die Gruppe mit der Anwendung des zu untersuchenden Präparats der Gruppe mit positiver Kontrolle (Hypothermie) und der Gruppe der falsch operierten Tiere nahe.

Bei der Analyse der Verhaltensangaben der Tiere wurde in der Anlage «Offenes Feld» der ausgeprägte Einfluss der intraperitonalen Einführung der Kreatin-Amide auf einen der Kennwerte des funktionellen Zustands der Tiere bei chronischer PPM festgestellt, nämlich die Latenz der Bewegungsauslösung, welche den Störungsgrad der Neuronstruktur und der Interneuronverbindungen wiedergibt. Es wurden die Daten in Bezug auf die Latenz der Bewegungsauslösung der Ratten aus der Gruppe mit intraperitonalen Injektionen der Zusammensetzung (n=7), der negativen Kontrolle (n=13), der positiven Kontrolle (n=5) und der falsch operierten Tiere (n=5) ein Tag vor und 1, 3 und 7 Tage nach der PPM verglichen.

Die Bewegungsauslösung (warm-up) ist die komplexe mehrstufige Bewegungsreaktion des Körpers auf innere bzw. äußere Anreize.

In Übereinstimmung mit der von Golani et al (Golani I, Wolgin DL, Teitelbaum, P., 1979) vorgeschlagenen Prozedur wurde zur Beurteilung der Bewegungsauslösung die Zeit gemessen, welche das Tier benötigt, um die betrachtete Raumfläche - Quadrat 20 × 20 cm - zu verlassen, welche im Mittelpunk der Anlage "Offenes Feld" liegt (1 × 1 × 0,5 m).

Die ermittelten Ergebnisse sind in der Tabelle 5 angeführt.

**Tabelle 5**

| Angaben der Verhaltenstests der Tiere im «offenen Feld» (Zusammensetzung 2, Versuch a) | | | | |
|---|---|---|---|---|
| **Einwirkung** | **Latenz der Bewegungsauslösung, c** | | | |
| | 1 Tag vor Ischämie | 1 Tag nach Ischämie | 3 Tage nach Ischämie | 7 Tage nach Ischämie |
| PPM | 9±2 | 49±4 | 46±4 | 16±3 |
| PPM + Zusammensetzung 2 | 9±2 | 25±6 | 30±8 | 10±2 |
| PPM + Hypothermie | 8±2 | 10±8 | 5±2 | 2±2 |

Die Einführung der Kreatin-Amide verursacht eine sichere und nachweisbare Minderung der Schädigungsareale des Gehirns bei experimenteller Ischämie (Tabelle 6).

**Tabelle 6 - Kenndaten der Gehirnschädigung bei experimenteller Ischämie/ postischämischer Reperfusion im Hintergrund der Einführung der Kreatin-Amide**

| Präparat, Anzahl der Tiere (n) | Verhältnis der Schädigungsfläche zur Fläche des gesamten Gehirnvorderquerschnitts (%) |
|---|---|
| Kontrolle, physiologische Lösung, intrazerebroentrikuläre Einführung, Modell des Experiments (c), (n=6) | 17,0 ± 1,8 |
| Zusammensetzung 8, intrazerebroentrikuläre Einführung, Modell des Experiments (c), (n=5) | 10,3 ± 1,0 |
| Zusammensetzung 9, intrazerebroentrikuläre Einführung, Modell des Experiments (c), (n=5) | 11,9 ± 2,0 |
| Zusammensetzung 10 intrazerebroentrikuläre Einführung, Modell des Experiments (c), (n=6) | 8,4 ± 0,7 |

Die hier angeführten Angaben zeugen von der neuroprotektiven Wirkung der Präparate, welche die Kreatin-Amide enthalten, bei Ischämie des Hirngewebes. Die Zusammensetzungen auf der Basis der Kreatin-Amide wirken sich positiv auf die Wiederherstellung der kognitiven Funktionen nach experimentellem Insult sowie auf die Latenz der Bewegungsauslösung aus, welche den Gesamtzustand des energetischen Gewebemetabolismus des Hirngewebes des Tieres, den Funktionszustand der Neuronen und der Assoziationsgebiete der Hirnrinde kennzeichnen.

## Patentansprüche

1. Kreatin-Amide mit der Gesamtformel: NH=C(NH₂)-N(CH₃)-CH₂-CO-NH-R*X, wobei R ein Aminosäurerest einer aliphatischen, aromatischen oder heteroaromatischen Aminosäure oder ihr Derivat ist, welches pharmazeutisch geeignete Salze der Aminosäure, Ester der Aminosäure, Amide der Aminosäure oder Peptide darstellt und
X eine niedermolekulare organische Säure oder Mineralsäure oder Wasser ist.

2. Verfahren zur Herstellung der Kreatin-Amide nach Anspruch 1,
**dadurch gekennzeichnet,**
**dass** die Reaktion des guanidierenden Agens mit Amiden von Sarkosin in polaren organischen Lösungsmitteln bei einer Temperatur von max. 50° C durchgeführt wird.

3. Amide von Kreatin mit der Gesamtformel:
NH=C(NH₂)-N(CH₃)-CH₂-CO-NH-R*X,
wobei R der Aminosäurerest einer aliphatischen, aromatischen oder heteroaromatischen Aminosäure oder ihr Derivat ist, welches pharmazeutisch geeignete Salze der Aminosäure, der Ester der Aminosäure, der Amide der Aminosäure oder Peptide darstellt und
X die niedermolekulare organische Säure oder Mineralsäure oder Wasser ist, welches als Mittel mit neuroprotektiver Wirkung dient.
